# EUROPEAN PATENT APPLICATION

(11) **EP 2 706 357 A1**
(43) Date of publication of application: **12.03.2014**
(21) Application number: 12183492.3
(22) Date of filing: 07.09.2012
(51) Int. Cl.: G01N 33/574

(54) **Method for identifying subgroups of circulating tumor cells (CTCs) in a CTC population or a sample**

(71) Applicant: Hoffmann, Andreas-Claudius, 45239 Essen (DE); Schlaak, Jörg Friedrich, 45131 Essen (DE)
(72) Inventor: Hoffmann, Andreas-Claudius, 45239 Essen (DE); Schlaak, Jörg Friedrich, 45131 Essen (DE)
(74) Representative: Simandi, Claus

(57) **Abstract**

The invention relates to a method for identifying and characterizing subpopulations (subgroups) of circulating tumor cells (CTCs) in a population of CTCs or a sample. The invention also relates to the quantification of the respective subgroups of CTCs in the population of CTCs or the sample. The invention further relates to the use of the method for diagnosis and monitoring of cancer, treatment and prognosis, in particular for solid tumors. The invention further relates to the use of the identified CTC subgroups as biomarkers for diagnosis, prognosis and therapeutic treatment. In addition, the invention relates to diagnostic devices and test kits for application of the method of the invention.

## Description

The invention relates to a method for identifying and characterizing subpopulations (subgroups) of circulating tumor cells (CTCs) in a population of CTCs or a sample. The invention also relates to the quantification of the respective subgroups of CTCs in the population of CTCs or the sample. The invention further relates to the use of the method for diagnosis and monitoring of cancer, treatment and prognosis, in particular for solid tumors. The invention further relates to the use of the identified CTC subgroups as biomarkers for diagnosis, prognosis and therapeutic treatment. In addition, the invention relates to diagnostic devices and test kits for application of the method of the invention.

Hepatocellular carcinoma (HCC) currently is the fifth most common malignancy worldwide with an annual incidence up to 500 per 100000 individuals depending on the geographic region investigated. Whereas 80% of new cases occur in developing countries, the incidence increases in industrialized nations including Western Europe, Japan and the United States (El-Serag HB, N.Engl.J.Med. 1999; 340:745-750). To manage patients with HCC, tumor markers are very important tools for diagnosis, evaluation of disease progression, outcome prediction and evaluation of treatment efficacy. Several tumor markers have been reported for HCC, which include α-fetoprotein (AFP) (Di Bisceglie AM J Hepatol 2005), Lens culinaris agglutinin-reactive fraction of AFP (AFP-L3) (OKA H, J Gastroenteroll Hepatol 2001), and des- γ-carboxy prothrombin (DCP) (Liebman HA N Engl J Med 1984). However, none of these tumor markers show 100% sensitivity or specificity, which calls for new and better biomarkers.

In order to identify novel biomarkers of HCC, many clinical studies utilizing omics-based methods have been reported over the past decade. In particular, the proteomics-based approach has turned out to be a promising one, offering several quantification techniques to reveal differences in protein expression that are caused by a particular disease. In the most studies reported in literature, the well-established 2D-DIGE (two-dimensional difference in gel electrophoresis) technique has been applied for protein quantification followed by identification via mass spectrometry.

Curative therapeutic options are limited to early stages and include mostly resection, sometimes orthotopic liver transplantation (LTx) if patients present with cirrhosis (3, 4). High recurrence rates after resection and even liver transplantation, most likely due to minimal residual disease (5, 6) and the fact that most patients are diagnosed in an advanced stage make palliative approaches necessary. The treatment of choice in a palliative setting is local administration of chemotherapy or radiotherapy. A selective internal radiation therapy (SIRT) employing 90mYttrium-loaded microsphere has become a major part of this local approach (7, 8). Up to now there are only very few possibilities to monitor treatment success. CT-scans often need several weeks to show an effect of local therapies, leaving the patient at risk of progressing unnoticed and hence limiting further options due to the effect of progressing liver disease. Furthermore there are still no reliable markers of a relapse after primary tumor resection or LTx. Currently available serum based markers like Alpha-fetoprotein (AFP), Des-Gamma-Carboxyprothrombin (DCP) or the Lectin 3-Fraction of AFP (AFP-L3) are incapable of predicting the clinical outcome with high accuracy and reproducibility (9). Tissue derived molecular markers seem to be a promising approach to individualize patients' therapy but lack the possibility of monitoring the patient during or after treatment, since this would require ongoing biopsies and hence increased risks for the patient. Therefore the development of minimal invasive diagnostic methods is imperative to characterize the tumor at the molecular level, to find sensitive markers for monitoring of postoperative recurrence as well as to evaluate therapy response and consequently to give adequate treatment for HCC patients.

Circulating tumor cells (CTC) in the peripheral blood of patients with HCC may be the solution to this diagnostic dilemma as a so-called "liquid biopsy". They already have been described in breast and lung cancer (10-12). Up to now only a few reports have been published on the isolation of CTC in HCC patients using indirect methods like qRT-PCR or direct visualization of circulating epithelial cells (13-17). So far CTC detection methods consist of enrichment and subsequent identification, with immunomagnetic bead selection based on antibodies for tumor cell surface antigens being currently the most common method.

Epithelial cell adhesion molecule (EpCAM) is widely expressed on the surface of epithelial cells and epithelial derived tumor cells (18). Therefore EpCam positive CTC were quantified and correlated with patients' outcome to examine their use in the clinical setting (12, 19). Mahreswaran and colleagues showed that CTC were also applicable for molecular approaches during therapy like the detection of EGFR mutations in patients with Non-small cell lung cancer (20). Identification of circulating tumor cells is often done with anti-cytokeratin antibodies, since blood of healthy individuals contains few or no cytokeratin-positive cells (21). In recent years the epithelial-to-mesenchymal-transition (EMT) has gained more attention since tumor cells may downregulate both EpCAM and CK to gain invasiveness and metastatic potential and potentially resistance to ongoing systemic treatment (22, 23). Therefore focusing on epithelial cell surface markers is not sufficient for detecting a heterogeneous population of CTC including those with a mesenchymal phenotype.

The problem to be solved is to provide an easy and reliable marker and diagnostic method for cancer, in particular for solid tumors like for example HCC and non-small cell lung cancer.

The present invention provides a method for studying biological samples for tumors, e.g. for HCC or non-small cell lung cancer. The present invention provides a method to identify and charcterize circulating tumor cells (CTCs) in a biological sample and to align these CTCs to subgroups. Thereby the CTC subgroup composition of a biological sample can be determined and changes in the CTC subgroup composition of a sample e.g. from a particular patient can be detected. The CTC subgroups identified by the method of the invention can be used as biomarkers for cancer and tumors. The novel biomarkers of the inventions are subtpyes/subgroups of circulating tumor cells (CTCs) that are characteristic for example for the patient, the tumor, the stage and the aggressiveness of the tumor. The mixture of subgroups (subtypes) of CTCs in a population of CTCs or a sample is identified by the method and can be used as a reliable biomarker.

The CTCs comprised in a population of CTCs or a sample can be assigned to one or more different subgroups (subpopulations) of CTCs, e.g. CTCs that are in the epithelial stage or CTCs that are in the mesenchymal stage or CTC that display features of the epithelial stage and the menchymal stage. With the method of the invention the different CTC subgroups can be identified, this could be only one or two or several different subgroups of CTCs. In addition, the amount of different CTC subgroups in a population of CTCs or a sample can be determined be using the method. Furthermore, the ratio of different subgroups of CTCs in the population of CTCs or a sample can be determined and the changes in the ratio or amount can be monitored over time. The method according to the invention allows monitoring changes in the composition and/or the ratio of CTC subgroups in a population of CTCs or a sample.

The present invention relates to a method of identifying a subgroup of circulating tumor cells (CTC) in a population of CTCs or a sample comprising the steps
a) depleting hematopoietic cells from a sample,
b) testing the obtained cell suspension for the presence of one or more epithel marker(s),
c) testing the obtained cell suspension for the presence of one or more mesenchymal marker(s), and optionally
d) testing the obtained cell suspension for the presence of one or more marker(s) specific for the respective tissue and /or the respective tumor.

A sample can comprise a population of CTCs. A population of CTCs can comprise on or more subgroups of CTCs. The composition of subgroup(s) of circulating tumor cells (CTCs) is characteristic for a specific tumor, for example a specific solid tumor. CTCs subpopulations and the respective subgroups of CTCs are therefore surrogate markers for cancer, tumors and /or metastasis. The subgroups of CTCs are in addition charcteristic for a particular patient. The individual subgroup CTC composition in the sample is specific for a particular patient. It can be used to predict and monitor treatment and disease progression or regression respectively. The CTC subgroups and the respective amounts (ratio) in the population of CTCs or the sample that are identified by the method of the invention can therefore be used as specific biomarker for the respective tumor, the therapeutic treatment and the individual patient, these biomarkes are for example suitable for individualized medicine.

The method according to the invention can in addition comprise the step of isolating or enrichment of peripheral blood mononuclear cells (PBMC) from the population of CTCs or the sample, e.g. a blood sample. In a preferred embodiment of the invention the sample is a is blood sample, for example blood serum, blood plasma, whole blood, a biopsy sample. The sample than comprises many PBMCs which are separated from the rest of the cell suspension.

However, it is not necessary that the separation is complete. In preferred embodiment of the invention at least 50 % of the PBMCs of the original sample, e.g. the peripheral blood serum of the patient, are separated from the blood sample in order to obtain a cell suspension depleted from hematopoetic cells. In a preferred embodiment of the invention PBMCs are isolated or enriched by density gradient centrifugation.

In another preferred embodiment of the invention hematologic cells are depleted using anti-CD45 antibodies, for example beads coated with anti-CD45 antibodies. In the method according to the invention hematopoietic cells are depleted from the population of CTCs or the sample, e.g. the blood sample with anti-CD45 coated immunomagnetic beads. In a preferred embodiment a mixture of beads, preferably immunomagnetic beads, linked with CD45 and CD15 antibodies are used.

According to the invention for example 55 to 85 %, preferably 65 to 75 %, most preferred about 70 % of the hematopoietic cells are depleted from the population of CTCs or the sample, e.g. the blood sample.

In another preferred embodiment the method according to the invention comprises the step of enrichment of epithelial cells after the CD45-depletion step. The enrichment of epithelial cells in the cell suspension can be performed for example with anti-EpCAM antibodies. The anti-EpCAM antibodies are for example bound to immunomagnetic beads. Cells bearing EpCAM are bound to anti-EpCAM antibodies and can than easily be separated from the cell suspension be removing the immunomagnetic beads.

The epithel marker(s) is / are for example selected form the group comprising epithelial cell surface markers, epithelial cell adhesion molecule (EpCAM), epithelial growth factor recepctor (EGFR), cytokeratin (CK), pan-cytoceratin (Pan-CK), Hep-Par-1, cytoplasmatic intermediate filament protein (Krt18), ASGPR1, DAPI, AFP, IGFBP1, CD133, LTR. Preferably EpCAM is used as epithel marker.

Mesenchymal marker(s) is / are for example selected from the group comprising N-Cadherin, Vimentin.

In a preferred embodiment of the invention the markers (epithelial markers, mesenchymal markers, tissue markers, tumor markers) are detected by immunofluorescence. Different subpopulations (subgroups) of CTCs can than for example be detected by counter staining. Individual profiles of patients, of a particular tumor, of a particular stage of tumor, of tumor progression or regression, of monitoring of treatment are for example created by counter staining thereby detecting individual characteristic subgroups of one or more CTCs. The method according to the invention thereby allows the determination of the composition of the CTC population at a certain point of time. This can be used for example for prognosis and therapeutic strategy. In addition, the method is suitable to monitore the changes in the composition of the CTC population during time, for example during teatment or after treatment.

In another preferred embodiment of the invention the method comprises the step of testing the cell suspension/the CTCs for one or more markers for epithelial-to-mesenchymal-transition (EMT). Markers for EMT are for example N-Cadherin and/ or c-Met.

The invention further relates to a method according to the invention for identifying a subgroup of circulating tumor cell (CTC) associated with HCC comprising the steps
a) enrichment of mononuclearic cells including CTCs from a blood sample,
b) depletion of hematopoietic cells from the blood sample,
c) use of one or more of markers to identify HCC specific CTC subgroup(s), for example one or more of the markers selected from the markers Pan-CK, HepPar-1, EpCAM, Krt18, IGFBP1, HGFAC, Hilf1a, TGFb, APC, AFP, Ktr19, ASGPR1, Vimentin, DAPI and N-Cadherin.

The invention further relates to a method according to the invention for identifying a subgroup of circulating tumor cell (CTC) associated with non-small cell lung cancer comprising the steps
a) enrichment of mononuclearic cells including CTCs from a blood sample,
b) depletion of hematopoietic cells from the blood sample,
c) use of one or more of markers to identify non-small cell lung cancer specific CTC subgroup(s), for example one or more marker(s) selected from the markers Pan-CK, CD133 and N-Cadherin.

The invention provides a method for identifying and isolating different types of CTC in the peripheral blood of patients with cancer, in particular solid tumors, for example hepatocellular carcinoma. The distribution of CTC subgroups in the whole CTC population serves as a biomarker to predict treatment response or outcome. The CTC subgroups and CTC population bears the potential to develop molecular markers to individualize treatment of cancer, in particular solid tumors, for example HCC or non-small cell lung cancer.

A quantification of the CTCs in subgroups is for example possible using Immunofluoresenz-labelling or PCR. It therefore allows to dermine and monitore the amount and / or ratio of different subgroups of CTCs in a CTC population or a sample. The invention further relates to the use of the method according to the invention, for example for characterization of tumors, for example for characterization of hepatocellular carcinoma (HCC) and / or non-small cell lung cancer.

The invention relates to the use of the method for determining the amount of mesenchymal CTCs to epithelial CTCs in the CTC polpulation or the sample. The invention relates to the use of the method for liquid biopsy. The invention relates to the continous monitoring of patients and/or sample composition with respect to CTC subgroups.

The invention further relates to the use of the method for determining the ratio of mesenchymal CTCs to epithelial CTCs in the sample or the cell suspension respectively. The invention in this connection also relates to the use of the method for predicting the progression free survival. The aggressiveness of the tumor, the responsiveness to treatment and the prognosis can be determined by the ratio of mesenchymal CTCs to epithelial CTCs. A ratio of mesenchymal CTCs to epithelial CTCs (e.g. determined by cells expressing N-Cadherin+/CK+) lower than 0,5, preferably lower than 0,2, most preferred lower than 0,1 resembles for expampe an association to shortened progression free survival. Therefore, the invention further relates to the use of the method for predicting aggressiveness of tumor, progression free survival (PFS), disease control, selection of treatment, surveillance of treatment, response to treatment, outcome of treatment, metastasis and recurrence.

The invention further relates to the use of the method for determining the stage of epithelial-to-mesenchymal-transition (EMT) of the respective tumor.

The invention further relates to the use of the method for diagnosis. The invention further relates to a diagnostic device or a test kit for the analysis of tumor specific CTCs in a sample. The invention further relates to a diagnostic device or a test kit for the enrichment and detection of tumor specific CTCs. Such diagnostic test or test kit for performing a method according to the invention may comprising means for depleting hematopoietic cells from a blood sample and means for detection of one or more markers selected form epithelial markers, mesenchymal markers and / or tumor markers. A diagnostic device or test kit according to the invention my comprise means for the detection of the ratio of different subtypes of CTCs in a blood sample. A diagnostic device or test kit according to the invention may for example comprise means for the detection of EpCAM. A diagnostic device or test kit according to the invention may comprise means for the immunological detection of the respective markers of CTC subgroups, preferably for their immunfluorescence detection.

The invention further relates to a diagnostic device or test kit for analysing the amount of CTC subgroups comprising at least one agent for the specific detection of one or more marker(s) associated with EMT, epithelial stage, mesenchymal stage and the respective tumor and wherein the diagnostic device or test kit further comprises detection reagents and further aids.

The invention further relates to a diagnostic device or a test kit wherein the detection reagent comprises an antibody specific for the respective marker.

In another embodiment the invention relates to the use of one or more markers specific for epithel cells, mesenchymal cells and / or the respective tumor for differential diagnosis, in particular for early recognition, diagnosis, evaluation of disease progression, prediciton of outcome, evaluation of treatment, surveillance of treatment of cancer.

The present invention further relates to a method for studying a biological sample for the respective tumor, wherein the sample is studied for one or more marker(s) for epithel cells, mesenchymal cells and/or tumor and wherein the marker(s) is /are differentially expressed in relation to the healthy state indicating the presence of the tumor.

In one embodiment of the invention the method for studying a biological sample is **characterized in that** the sample is a human sample.

In one embodiment of the method the differential expression of the particular marker in the respecitive CTC (sub-)population is determined by comparing the amount of this marker in a biological sample of a person without the disease with the amount of this protein in a person with the disease.

The present invention further relates to the use of the CTC subgroups identified by the method according to the invention for screening pharmaceutical compounds for the respective tumor and / or the respective patient.

The present invention further relates to a screening assay comprising one or more CTC subgroups identified by the method according to the invention and using theses CTC subgroups for the identification and validation of pharmaceutical compounds for a respective tumor and / or a respective patient and wherein the screening assay comprises detection reagents and further aids.

In the context of this invention, the term HCC comprises any form of Hepatocellular carcinoma (HCC). The term is for example defined in Pschyrembel, Klinisches Wörterbuch [Clinical Dictionary], 263th edition, 2012, Berlin.

In the context of this invention, the term non-small cell lung cancer comprises any form of non-small cell lung cancer. The term is for example defined in Pschyrembel, Klinisches Wörterbuch [Clinical Dictionary], 263th edition, 2012, Berlin.

The invention also comprises the use of isoforms of the respective markers. An "Isoform" of the respective protein is any of several different forms of the same protein. Different forms of a protein may be produced from related genes, or may arise from the same gene by alternative splicing. A large number of isoforms are caused by single-nucleotide-polymorphisms or SNPs, small genetic differences between alleles of the same gene. These occur at specific individual nucleotide positions within a gene. Isoforms comprise also proteins with the same or similar amino acid sequence but different post-translational modification, like glycosylation. A glycoform is an isoform of a protein that differs only with respect to the number or type of attached glycan. Glycoproteins often consist of a number of different glycoforms, with alterations in the attached saccharide or oligosaccharide.

The invention also comprises homologes of the respective markers. A "homologe" of the respective protein is defined in terms of shared ancestry. Two segments of DNA can have shared ancestry because of either a specitation event (orthologs) or a duplication event (paralogs). The term "percent homology" and "sequence similarity" are used interchangeably. High sequence similarity might occur because of convergent evolution or because of chance. Such sequences are similar and are also included in the term according to the invention. Sequence regions that are homologous are also called conserved. Enclosed is also partial homology where a fraction of the sequences compared (are presumed to) share descent, while the rest does not. Many algorithms exist to cluster protein sequences into sequence families, which are sets of mutually homologous sequences, see for example databses HOVERGEN, HOMOLENS, HOGENOM. According to the invention homologes should display at least 80 % or 90 % or 95 % identity in amino acid sequence with the marker, preferably 96 % or 97 %, most preferably 98 % or 99 %.

With this invention a method was established to examine whether circulating tumor cells (CTC) and subtypes are identifiable in the peripheral blood of patients with cancer, in particular hepatocellular carcinoma.

Two strategies are applied to enrich and detect CTC in cancer, e.g. HCC. By one strategy CTC were negatively enriched by depleting hematopoietic cells using anti-CD45 immunomagnetic beads leading to a bead-free cell suspension. This cell suspension was then used for CTC type detection by immunofluorescence staining against various epithelial (e.g. Pan-CK, EpCAM) and mesenchymal markers (e.g. Vimentin, N-Cadherin) and one or more tissue specific marker(s), for example the liver specific ASGPR1.

Concomitant an equal part of this solution was further enriched after the depletion step using anti-EpCAM immunomagnetic beads resulting in a bead-bound EpCAM-positive CTC suspension. Identification of CTC in the EpCAM-positive bead-bound cell suspension was performed using immunocytochemical staining against Pan-CK or Hep Par 1, respectively with alkaline phosphatase. The CTC-enrichment step was confirmed by measuring mRNA expression levels of the epithelial genes EpCAM and Krt18 in depleted and enriched fractions.

To assess whether it is possible to identify and evaluate prognostic factors the genetic profile of CTCs in blood from patients during SIRT1 was analyzed. The prognostic values of several candidate markers involved in carcinogenesis and liver disease, namely IGFBP1, HGFAC, Hif1a, TGFb, APC, AFP, Hif1a and Krt19 as well as their interrelationships was analyzed. The association of these gene expressions in EpCAM-positive CTC with response to SIRT using quantitative real-time RT-PCR in 25 patients with either disease control (partial response & stable disease) or progression was analyzed.

Therefore, two strategies were employed to enrich and detect CTC in HCC. Firstly, CTC was negatively enriched by depleting hematopoietic cells using anti-CD45 immunomagnetic beads leading to a bead-free CTC suspension which was used for CTC detection by immunofluorescence staining. Different populations of CTC were detected, such as epithelial cells that stained positive for pan-CK and DAPI but negative for EpCAM and CD45 as well as cells staining positive for both CK and EpCAM while negative for CD45. Also CTCs with mesenchymal properties such as vimentin and N-Cadherin as well as cells with both epithelial and mesenchymal features were detected. It has been described by Kalluri et al that CTC might undergo EMT and subsequently lose their epithelial features such as cell surface marker EpCAM and CKs (23). The detection of CTC showing mesenchymal phenotype was also reported by Gradilone et al. who assessed the prognostic value of CTC expressing mesenchymal markers in metastatic breast cancer patients (25, 26). They characterized CTC for CK and markers of EMT and found the gain of mesenchymal markers in CTC to be correlated to prognosis of patients in a follow-up of 24 months. Their data showed that the presence of mesenchymal markers on CTC more accurately predicted worse prognosis than the expression of CKs alone.

The data in the examples shows that the presence of mesenchymal cells correlates to survival. However the results indicated that an increase in mesenchymal cells associates with better treatment outcome - hence that epithelial cells seem to be the driver of aggressiveness in this study population. Supporting these findings in the tested patients with HCC it was also revealed that the total amount of CK+ cells was higher in patients with a worse Child status. These contradicting results may be explainable by the different entity (breast vs. hepatocellular cancer) or different treatment approaches. Nearly all patients in this study had epithelial and mesenchymal cells in the peripheral blood, but in different proportions. Only some of these patients (4/11; 36%) presented cells with both features on the same cell. This indicates different stages of EMT. Cells were detected which stained positive for pan-CK and CD45 as well as for EpCAM, pan-CK and CD45. Cells with epithelial and hematological features were already described by Yu et al. (24). Since the identity of those cells is not yet clear they are generally excluded from the cell count in the clinically used methods, such as the CellSearch System (Veridex). Nevertheless, this approach holds potential bias, since it has not yet been shown that these cells with additional CD45+ staining really are hematopoietic. Especially since the transfer of membrane proteins during contact known as trogocytosis can create novel cells with unique phenotype and altered function (27). In addition to this immunocytological variety of CTC distinct differences in size were observed. Commonly, CTC are described to have a large cellular size and a high nuclear to cytoplasmic ratio (14). In this study a wide range of signal intensities and a variety of cell sizes was found. There are a few reports though which confirm a significant variation of size within the same sample and different nuclear to cytoplasmic ratios (21, 28). Isolation by size of epithelial tumor cells (ISET) was applied by Vona et al. in patients with primary liver cancer (PLC) in order to evaluate its clinical impact. Fifty-two percent of total 44 PLC patients had detectable CTC, and their prevalence was significantly correlated with the presence of multifocal tumors, portal tumor thrombosis, and Child-Pugh class B/C (15). Hofman et al. compared ISET and CellSearch system with blood samples from NSCLC patients. Double immunostaining of the filters against CK and vimentin after ISET revealed circulating cells with non-hematopoietic features, but with cytological malignant criteria, which expressed only vimentin in a subpopulation of patients with no CTC detected by the CellSearch system (29).

The CellSearch system was applied by Schulze et al. to detect EpCAM-positive CTC in blood of HCC patients resulting in a detection rate of 45% (14 out of 31 HCC patients) and a significant correlation between the detection of CTC and pathological AFP values.(17) Xu et al. reported the development and validation of an EpCAM-independent magnetic cell separation system mediated by the interaction of the asialoglycoprotein receptor (ASGPR1) exclusively expressed on hepatocytes with its ligand. CTCs were then identified by Hep Par 1 staining. An average of 24 ± 19 CTCs per 5 ml blood was detected in 81% of HCC patient.

In the present invention negative CTC isolation was combined with immunofluorescence detection. Although hematologic cells were depleted using Anti-CD45 beads many cells remained in the suspension (log order 2 according to manufacturer's protocol; CD45 levels differ in leucocytes). The depletion rate was about 70% (data not shown). This method does not entirely deplete hematologic cells, but it offers an opportunity to detect the cells in a sample and to distinguish between different cell populations via counter staining and create an individual profile of each patient which might help anticipate patients' outcome. Since it is based on basic principles such as cellspin and immunolabeling it is an inexpensive procedure allowing quick clinical implementation. It is a cell size-independent method that includes, but is not solely dependent on EpCAM. The method of the present invention recognizes the fact that the total amount of hematopoietic as well as tumor cells varies significantly between patients and during treatment courses. Though the main goal of this study was to identify different CTC types, a ratio of these subtypes that seems to correlate well with therapeutic response was also established.

In order to extend the range of possible tumor cell enrichment it is necessary to have a system with greater flexibility to enrich and detect additional types of CTCs. The EpCAM-based enrichment method after density gradient centrifugation was comprehensively assessed by Guo et al who spiked healthy blood with a serial dilution of HepG2 cells and reported that as little as 10 cells in 5 ml blood could be detected using RT-PCR with primers specific for AFP. In clinical samples the positive detection rate ranged from 53 to 93% depending on the Child Pugh class (A, B and C) with a total positive detection rate of 73% (16). In the present study epithelial tumor cells seemed to associated with worse outcome and more aggressive disease course. Therefore epithelial cells were further enriched after the CD45-depletion step using anti-EpCAM immunomagnetic beads resulting in a bead-bound EpCAM-positive CTC suspension which still contained hematologic cells due to incomplete depletion. EpCAM- and KRT18 mRNA expression levels were compared in all cell fractions and was found to be significantly increased in the EpCAM-positive cell suspension compared to the CD45-positive hematopoietic cell fraction. These data revealed a specific enrichment of EpCAM-positive CTC employing anti-EpCAM immunomagnetic beads combined with a CD45 depletion step. Interestingly, KRT18 expression was significantly increased in the remaining bead-free cell suspension after CD45-depletion and after withdrawal of EpCAM-positive cells compared to the hematologic as well as to the EpCAM-positive cell fractions. The mRNA expression analysis data confirmed the occurrence of EpCAM-negative/KRT18-positive CTC that remain unbound in the residual cell fraction highlighting the results of the immunocytological staining. Consequently the EpCAM enriched cell suspension was tested for potential molecular markers of therapeutic outcome choosing a set of genes involved in carcinogenesis and liver diseases.

In this invention a matched set of patients undergoing SIRT with either progression or partial response was used. The implementation of Spearman's test and the multivariate analysis illustrated a significant association of IGFBP1 (p=0.006) and AFP (p=0.04) mRNA expression with progression after SIRT (p=0.01). In this patient cohort however, AFP was excluded from the model when tested together with IGFBP1 mRNA expression (model fit p=0.01). After converting the continuous data to low and high expression groups with either progression or partial response (stable disease), low expression of IGBFP1 (p=0.01) and serological Bilirubin (p=0.02) were the only factors in this patient cohort showing a significant independent correlation to response. High AFP mRNA expression (p=0.0566) as well as serological AFP (p=0.0792) were by trend significant and Bilirubin (p=0.052) was significantly correlated to response. Interestingly, decreased IGFBP1 expression had a higher sensitivity (80%) and specificity (80%) than high AFP mRNA expression which has been reported as a well-recognized biomarker correlating with the metastasis and recurrence of HCC, and discussed to be the most useful molecular marker to prefigure the prognosis so far (16, 30). To validate the findings a second matched set of 11 HCC patients and repeated IGFPB1 mRNA expression analysis as described above was used. Spearman's test and the multivariate regression confirmed a significant correlation of IGFBP1 with progression after SIRT. The findings are in line with a study by Gong et al which revealed that IGFBP-1, 3 and 4 mRNA expression levels were significantly down regulated in HCC when compared to adjacent cirrhotic tissues and healthy livers which supports the concept that IGFBPs play an important inhibitory role in the development and/or growth of hepatocellular carcinoma (31).

In the present study IGFBP1 was also significantly associated with progression free survival in the Kaplan-Meier log rank test. ROC curve analysis indicated that changes in IGFBP1 mRNA expression ratios between early time points may help to evaluate therapy response or failure. Especially in liver cancer patients that do not benefit from treatment (e.g. SIRT) lose valuable time until re-evaluation of response using available imaging modalities.

With this invention different CTC populations are identifiable in peripheral blood of patients with HCC and it was shown that this individual cell type profile may have distinct clinical implications. Furthermore the data suggests that epithelial characteristics are associated with more aggressive tumors. The significant association of IGFBP1 mRNA expression to response in this study supports the use of CTC as biomarkers.

The following examples and figures are used to explain the invention without restricting the invention to the examples.

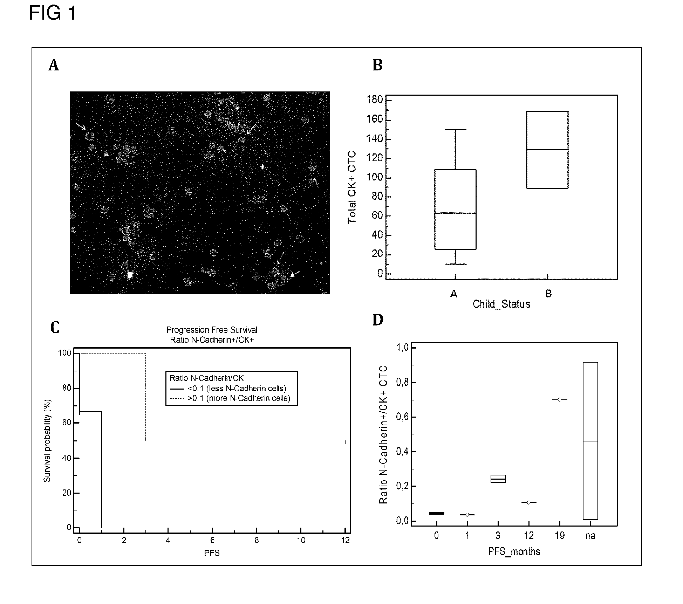

Fig. 1: Quantification of CTC subtypes and correlation with clinical characteristics. (A) Example of individual morphological profile showing 1 epithelial (CK-positive) and 3 mesenchymal (N-Cadherin-positive) CTC. (B) Amount of CK+ cells is associated to Child status. (C) Progression free survival vs. survival probability. (D) Ratio N-Cadherin+/CK+ cells is correlated to PFS (p=0.027).

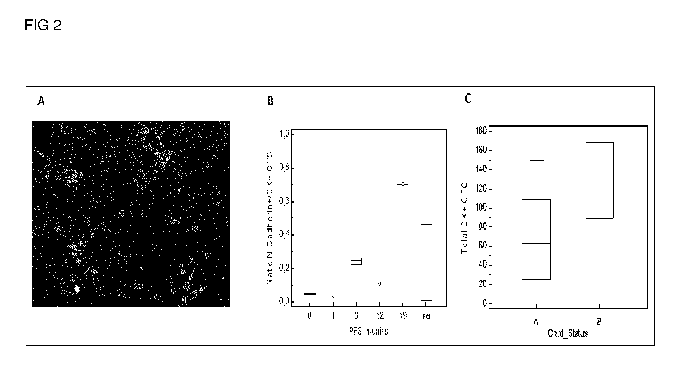

Fig. 2: CTC-enrichment. Detection of anti-EpCAM bead-bound CTC using immunochytochemical staining against (A) Pan-CK and (B) Hep-Par 1 with alkaline phosphatase. Gene expression levels of (C) EpCAM and (D) KRT18 in anti-EpCAM-bead-bound cells (EpCAM+), depleted hematologic cells (CD45+) and remaining bead-free cell suspension (Remain).

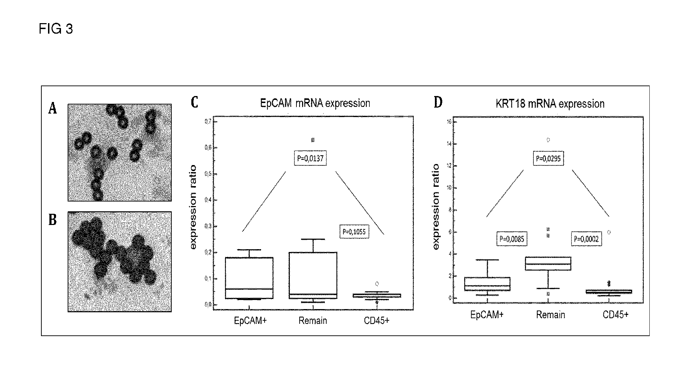

Fig. 3: Quantification of CTC subtypes and correlation with clinical characteristics. (A) Example of individual morphological profile showing 1 epithelial (CK-positive) and 3 mesenchymal (N-Cadherin-positive) CTC. (B) Ratio N-Cadherin+/CK+ cells is correlated to PFS (p=0.027). (C) Amount of CK+ cells is associated to Child status.

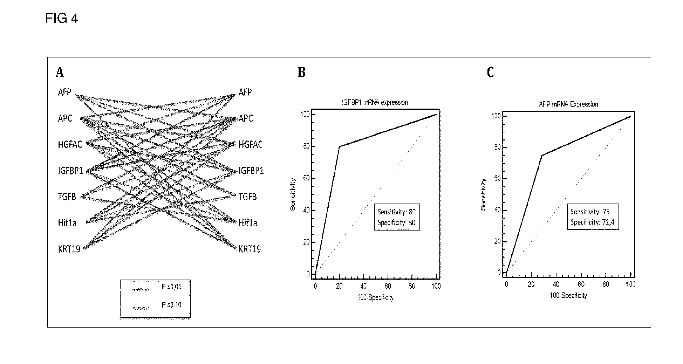

Fig 4: (A) Significant correlations between gene expressions measured in HCC patients. (B) ROC-curve analysis of IGFPB1 and (C) AFP.

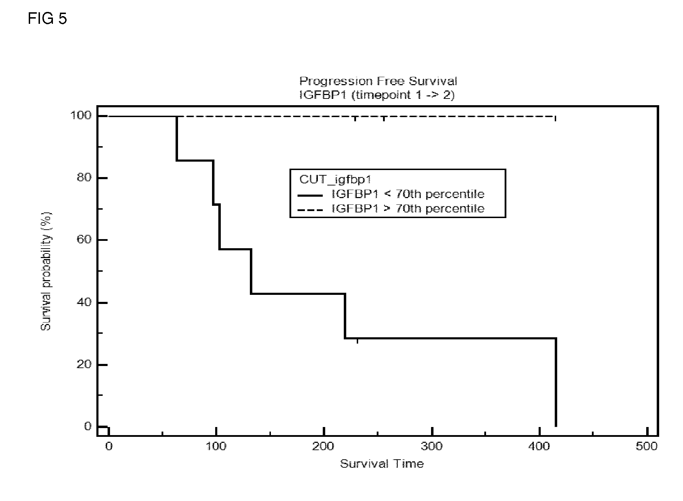

Fig. 5: Progression free survival vs. Survival probability.

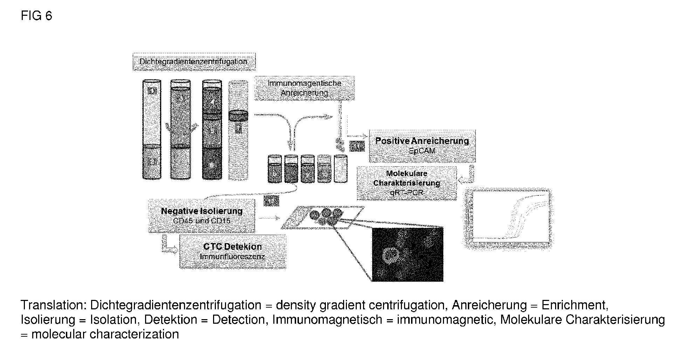

Fig. 6: Method according to the invention, schematic view.

### Examples

### Example 1: Materials and Methods

### Example 1a: Blood sample collection

40 ml citrated blood from HCC patients were collected, stored at room temperature and processed within 24 h after collection. Duplicates of 20 ml were used for sample preparation for subsequent CTC detection and gene expression analysis.

### Example 1b: Isolation of peripheral blood mononuclear cells (PBMC) using density gradient centrifugation

20 ml of citrated peripheral blood were diluted with 10 ml PBS and poured into a Leucosep tube (Greiner Bio-One) prepared with 16 ml Ficoll-Paque (GE-Healthcare) below the porous barrier. After density gradient centrifugation at 1600xg at 20°C for 20 min without brake the PBMNC containing interphase above the barrier was transferred into a new tube, washed with 50 ml PBS containing 0.5% BSA and centrifuged at 300xg at 20 °C for 10 min. Subsequently, supernatant was removed completely.

### Example 1c: Sequential tumor cell enrichment using immunomagnetic beads

Negative isolation: Washed PBMC were resuspended in 1 ml of PBS containing 0.1% BSA and incubated with 25 µl anti-CD45 coated immunomagnetic Dynabeads (Invitrogen) for 30 min. Hematopoietic cells except erythrocytes, platelets and their precursor cells were bound to beads and separated by a magnetic particle processor (King Fisher mL, Thermo Fisher). The remaining cell suspension included bead-free pre-enriched tumor cells and was either subjected to immunocytochemical staining (see below) or subsequent positive epithelial tumor cell isolation.

Positive isolation: Cell suspension was incubated for 30 min with 50 µl FCR-blocking reagent (Miltenyi) and 10 µl immunomagnetic Epithelial Enrich-Dynabeads (Invitrogen) coated with a mouse IgG1 monoclonal EpCAM antibody. Tumor cells that are expressing EpCAM on their cell surfaces (EpCAM+ CTC) were bound to beads, separated by the magnet, washed and transferred into a new tube containing 100 µl PBS.

Positive and negative isolated CTC enriched suspensions were spun onto two glass slides, respectively, using a cytocentrifuge (Cell Spin II, Tharmac). After air drying cells were fixed in 96% Ethanol for 10 min and stored at 4-8°C until stained. For gene expression analyses a second sample was prepared in the same manner, but the CTC enriched suspension was centrifuged for 3 min at 350xg. After removing the supernatant cells were lysed with RLT lysis buffer (Qiagen) and samples were stored at -80°C until RNA extraction.

### Example 1d: Identification of CTC subtypes after negative isolation using immunocytochemical labeling

Immunofluorescence staining of epithelial and hematopoietic cells was carried out in the CD45-depleted pre-enriched tumor cell suspension. Briefly the staining method includes
a) fixation of the cells in 4,5% formaldehyde for 15 min, wash in PBS ,
b) permeabilization with 1x Perm/Wash Buffer (BD Biosciences) for 10 min, wash in PBS,
c) blocking of unspecific antibody reaction by incubation with blocking solution containing 5% BSA for 30 min,
d) binding of primary antibodies either ASGPR1 rabbit monoclonal antibody (ab42488, abcam) or pan-cytokeratin guinea pig polyclonal antibody (ABIN126062, antibodies-online) and EpCAM (E144) rabbit monoclonal antibody (ab32392, abcam) or vimentin (EPR3776) rabbit monoclonal antibody (2707-1, Epitomics) or N-Cadherin (EPR1792Y) rabbit monoclonal antibody (2019-1, Epitomics) for CTC and anti-CD45 (MEM-28) mouse monoclonal antibody (ab8216, abcam) for hematologic cells overnight at 4°C, wash in 0,1% Tween,
e) binding of secondary antibodies (FITC-conjugated AffiniPure goat anti-rabbit and Cy3-conjugated AffiniPure goat anti-mouse or AlexaFlour647-conjugated AffiniPure F(ab')2 Fragment goat anti-guinea pig; Jackson ImmunoResearch) for 30 min at 37°C, wash in 0,1% Tween.

Subsequently, cells were stained with 4 ,6-Diamidino-2-phenylindole dihydrochloride (DAPI) (Sigma-Aldrich) for 10 min, mounted with anti-fading medium (Invitrogen) and stored in the dark until evaluation. For each test a positive control slide with a mixture of human PBMNC and the hepatocellular carcinoma cell line HepG2 was treated under the same conditions. Microscopic evaluation was carried out using the digital Keyence BZ9000 (model BIOREVO) all-in-one fluorescence microscope with integrated camera and BZ-Analyzer Software. Stained slides were manually examined and objects that stained positive for DAPI and pan-cytokeratin or EpCAM or ASGPR1 or vimentin or N-Cadherin and negative for CD45 were captured and considered as tumor cells.

### Example 1e: Quantification of CTC subtypes

After negative CTC isolation each sample was split in half and processed onto glass slides for N-Cadherin/CK/CD45 and vimentin/CK/CD45 staining, respectively. Subsequently CTCs were detected within the same areas, each consisting of 10 visual fields using a 20x magnification on both slides.

### Example 1f: Immunocytochemical staining for pan-cytokeratin-positive and Hep-Par-1-positive cells after EpCAM-positive tumor cell enrichment.

Detection of epithelial cells with the aid of immunofluorescence staining is technically impossible in the positively enriched CTC suspension due to light reflection by immunomagnetic beads. Therefore, identification of epithelial cells in the bead-bound enriched tumor cells suspension was performed using the UltraVision Quanto detection system AP (Thermo Scientific) according to the manufacturer's protocol. Briefly the staining method includes
a) permeabilisation with Perm/Wash buffer (BD Bioscience),
b) blocking of nonspecific background staining,
c) binding of primary mouse monoclonal pan-cytokeratin antibody-1 (Thermo Scientific, MS-343-R7) or Hep-Par-1 (Thermo Scientific, MS-1810-R7)
d) incubation with primary antibody amplifier Quanto (TL-125_QPB),
e) incubation with alkaline phosphatase-labeled polymer Quanto (TL-125-QAL) directed against mouse and rabbit antibodies, f) visualization of polymer complex with Liquid Fast Red (LV, TA-125-AL) substrate buffer and chromogen,
g) counterstaining with Mayer's haematoxylin ( TA-125-MH).

For each test a positive control slide with the HepG2 carcinoma cell line was treated under the same conditions. Identification of epithelial (CK+) cells was performed using the Leitz Dialux 20 EB microscope connected to a Leica DC 300F camera.

### Example 1g: Cell culture

Human carcinoma cell lines HepG2 (liver), HCT116 (colon) and A549 (lung) were obtained from the American Type Tissue Collection (ATTC) and maintained in culture flasks at 37°C with 5% CO₂ in a humidified atmosphere. Cells were grown in Dulbecco's Minimum Essential Medium High Glucose with L-Glutamin (PAA) containing 10% FBS (Biochrom AG) and 1% gentamycine (CC-Pro). Media for HepG2 cell line was additionally supplemented with 1% sodium pyruvate (PAA) and 1% nonessential amino acids (PAA). Cells were detached from the bottle by trypsinising.

### Example 1h: Clinical characteristics of patients for CTC quantification & gene expression analysis.

Within this invention patients' blood was drawn consecutively during treatment visits in outpatient unit. The clinicopathological data of the patients used for CTC quantification in this pilot study is listed in Table 1. Clinicopathological data of patients used for identification of molecular markers out of the positively enriched EpCAM+ CTCs is shown in Table 2. All blood samples were collected before or during treatment in intervals of 7 days for the first 3-4 drawings and later in case of follow-up visits in our outpatient unit. In the expression analysis prove-of-principle study and validation group, blood was also accepted for testing when drawn shortly after SIRT respectively planned time-points.

### RNA extraction

Total RNA was extracted from recovered EpCAM-positive tumor cells and from human carcinoma cell lines using MagAttract RNA Cell Mini M48 Kits (Qiagen) and King Fisher mL magnetic particle processor (Themo Fisher) according to the manufacturer's protocol. Subsequently, remaining DNA was removed using RQ1 RNase free DNase (Promega) according to the instruction supplied.

### Real time RT-PCR

One-step real time RT-PCR (Roche LightCycler 480) was performed using Quanti Fast SYBR Green RT-PCR Kits and QuantiTect primer assays (Qiagen) for the following genes: TGFb1 (QT00000728), HGFAC (QT00000077), HIF1a (QT00083664), KRT19 (QT00081137), KRT18 (QT01846327) AFP (QT00085183), APC (QT00038962), IGFBP1 (QT00049427) and EpCAM (QT00000371). The primers for reference gene b-actin (Eurofins MWG) were as follows: forward: 5'-GAGCGCGGCTACAGCTT-3', reverse: 5'-TCCTTAATGTCACGCACGATTT-3'. Assays were performed in triplicates to determine expression levels. Thermal cycling conditions were 10 min at 50°C and 5 min at 95°C for RT and initial denaturation followed by 50 cycles of 95°C for 10 sec and 60°C for 30 sec. Triplicates of HepG2- HCT116- and A549-RNA (10ng/µl) were used as internal standard to control each run. Each primer has been validated in a serial dilution of RNA extracted from cell lines mentioned above.

### Example 1i: Statistical Analysis

Spearman's rank correlation was used to examine associations between CTC subtypes and clinical characteristics of the patients. Associations of the cell type ratios and progression-free survival (PFS) were tested by the Kaplan-Meier method. Survival differences between patients with a high- and low-cell type ratio were analyzed by the log-rank test. For gene expression analyses Wilcoxon signed rank test was used to assess whether expression levels differ in CTC enriched and hematologic cell fractions.

The associations among gene expression levels and clinicopathologic parameters and between each gene expression were tested with Spearman test for bivariate correlations. To detect independent prognostic factors associated with progression each gene was tested in a linear backward multivariate regression model along with established clinical tumor markers AFP, Bilirubin and LDH with progression as dependent variable. Gene expressions that showed significant correlation to therapy response were then split in high- and low-level groups by setting a cut-point at the 50^{th} percentile Receiver operating characteristic (ROC) curve analysis was used to test the ability of the chosen cutoffs to discriminate progression from partial response (stable disease). The level of significance was set to P<0,05. All P values were based on two-sided tests. All statistical analyses were performed using the Software Packages SPSS for Windows (Version 19.0; SPSS, Inc, Chicago, IL) and Medcalc, Version 12.3.0 (Mariakerke, Belgium).

### Example 2: Results

### Immunocytochemical Identification of CTC subtypes

Objects that stained positive for DAPI and pan-cytokeratin, EpCAM or ASGPR1 and negative for CD45 were captured and considered as tumor cells. Pan-CK+/EpCAM+/CD45- as well as pan-CK+/EpCAM-/CD45- cells were detected after negative isolation. Furthermore pan-CK+ cells were detected in the remaining bead-free cell suspension after CD45 depletion and removal of EpCAM-positive CTC supporting the occurrence of a CK+/EpCAM- CTC subpopulation. Furthermore cells with mesenchymal features such as N-Cadherin+/CD45- and Vimentin+/CD45- were detected after negative isolation. Cells showing both epithelial and mesenchymal characteristics such as N-Cadherin+/CK+/CD45- and vimentin+/CK+/CD45- were also found. Cells that stained positive for potential markers of circulating tumor cells and CD45 were also enumerated.

### Subtypes and clinical outcome

Spearman's rank test resulted in a significant association of progression free survival (PFS) with the ratio N-Cadherin+/CK+ (p=0.04) and the ratio vimentin+/CK+ (p=0.02). Both mesenchymal/epithelial CTC ratios were by trend significantly correlated to each other (p=0.07). Tumor size was significantly correlated to therapy response (p=0.03) but displayed a trend to significance in correlation to the ratio N-Cadherin+/CK+ (p=0.1). The amount of epithelial CK+ cells and Child status also correlated by trend towards significance (p=0.1). To test whether the N-Cadherin+/CK+ ratio or the Vimentin+/CK+ ratio potentially divide patients according to their chance of a prolonged PFS Kaplan-Meier Survival Analysis was used. The Kaplan-Meier log rank test showed that both ratios are significantly associated with PFS. A Vimentin+/CK+ ratio higher than 0.5, meaning that there were half as many Vimentin positive cells then CK+ cells was associated with a longer median PFS (2 months, p=0.01; [HR]=0.18). A N-Cadherin+/CK+ ratio lower than 0.1 resembled an association to shortened progression free survival (p=0.03; [HR]=0.19).

### Identification of pan-cytokeratin-positive and Hep-Par-1-positive CTC after sequential positive enrichment.

The enrichment of epithelial cells using anti-EpCAM immunomagnetic beads was confirmed by immunocytochemical staining against pan-CK or Hep-Par-1, respectively. By microscopic observation, the EpCAM+ enriched (bead-bound) cell suspension contained epithelial cells that stained positive for pan-CK or Hep-Par-1 among hematopoietic cells. Due to shearing forces between beads, magnet and cells the membrane may sometimes rupture and cytoplasm leaks which leads to pink background staining. Results were reviewed by a clinical pathologist.

### mRNA expression of EpCAM and KRT18 in depleted and enriched cell suspensions.

To confirm the depletion step using anti-CD45 coated immunomagnetic beads and the subsequent CTC enrichment step using anti-EpCAM coated beads the mRNA expression of epithelial genes EpCAM and KRT18 in the CD45+, EpCAM+ and the remaining bead free cell fractions was examined. Expression levels of the epithelial cell surface marker EpCAM and the cytoplasmic intermediate filament protein KRT18 were significantly (p=0.0137 resp. p=0.0295) higher in the bead-bound EpCAM+ CTC enriched fraction compared to the CD45+ selected cell suspension. The EpCam mRNA expression was decreased (p=0.1) in the CD45+ selected hematopoietic cell fraction compared to the remaining bead-free cell fraction. EpCAM mRNA expression was slightly decreased in the remaining bead-free cell fraction compared to the EpCAM+ CTC enriched suspension. Comparing the EpCam+ respectively CD45+ selected cell suspensions with the remaining cell suspension KRT18 mRNA expression was significantly elevated (p=0.009 resp p=0.0002).

### PCR-based identification of potential markers of progression (SIRT).

The statistical associations of the abovementioned genes was tested, known clinicopathological parameters such as AFP, LDH and Bilirubin as well as treatment outcome with Spearman test. Progression was significantly correlated to serological tumor markers AFP (p= 0.04) and LDH (p= 0.003). Krt19 mRNA expression was significantly correlated to progression (p=0.05) and serological bilirubin (p=0.02, Spearman rank correlation coefficient: r=0.7). APC respectively AFP gene expressions were significantly correlated with bilirubin (p=0.05 r=-0.6 resp. p=0.03, r=0.6). Expression levels of Hif1a mRNA (p=0.05, r=0.6) and HGFAC (p=0.04, r=0.6) were significantly correlated to serological AFP whereas IGFBP1 (p=0.03, r=-0.7) and TGFB (p=0.03, r=-0.6) mRNA expression significantly but inversely correlated with LDH. IGFBP1 mRNA expression was significantly associated to AFP (p=0.0065, r=0.9), APC (p=0.0217, r=0.7), HGFAC (p=0.0125, r=0.8) and KRT19 (p=0.0053, r=0.8) and showed by trend significant correlation to Hif1a (p=0.0667, r=0.6.

Additionally, the changes of mRNA expression levels between time points in patients with progression versus disease control were investigated. Gene expression levels of AFP, HGFAC, TGFb, Hif1a ,and APC showed the largest changes between first and third, respectively second and third time point, whereas IGFBP1 mRNA expression showed the biggest differences between first and second time-point (p=0.06). Each gene was tested as a continuous variable in separate linear backward multivariate regression models along with established clinical tumor markers AFP, Bilirubin and LDH including progression as dependent variable. IGFBP1 mRNA expression showed the highest independent significant correlation to response (p=0.0062; model fit p=0.012) followed by HGFAC (p=0.0178; model fit p=0.008) and AFP (p=0.04; model fit p=0.008). TGFb and APC mRNA expressions showed tendency to significance at third time point (p= 0.1117; p= 0.1325)..AFP was excluded from the model when tested together with IGFBP1 mRNA expression (model fit p=0.01).

Patients were than split into low and high expression groups with either progression or partial response (stable disease) by choosing the cut-points at the 50^{th} percentile. To test whether gene expression levels were significantly different in-between subgroups the linear backward multivariate regression models including AFP, Bilirubin and LDH was repeted. Low expression of IGBFP (p=0.01) and serological Bilirubin (p=0.02) were the only factors in this patient cohort showing a significant independent correlation to response. The overall model fit had a significance level of p=0.016 (<0.05). High AFP mRNA expression (p=0.0566) as well as serological AFP (p=0.0792) were by trend significant and Bilirubin (0.052) was significantly correlated to response (over-all model fit of p=0.011).

An independent set of patients with similar clinicopathological characteristics (Table 2) was used as a validation set and assessed the IGFBP1 mRNA expression following the procedure described above. Spearman's rank test confirmed significant correlations of IGFBP1 expression with serological tumor markers LDH (p=0.03) and Bilirubin (p=0.01). Again a decrease of IGFBP1 expression during course of therapy was significantly associated with progression (p=0.0134; model fit p=0.015).

Kaplan-Meier log rank test was used to examine the association of the identified change in IGFBP1 expression between time-points and progression free survival. A decreased IGFBP1 expression significantly correlated to shortened PFS (p=0.04). These results were confirmed by Cox multivariate regression analysis (p=0.005; HR=0.02; data not shown due to low patient numbers) High IGFBP1 expression showed sensitivity (true positive rate) of 80% and specificity (true negative rate) of 80% for the diagnosis progression versus disease control. The area under the curve was 0.8 (CI 0.44 to 0.98) with a significance level of p = 0.03.

### Example 3: Circulating CD133+ stem and mesenchymal cells in peripheral blood of a patient with c-Met positive non-small cell lung cancer treated with Cisplatin/Pemetrexed (CisPem).

It has already been discussed that circulating tumor cells (CTC) may have potential as an easily accessible surrogate marker for therapeutic decisions in patients with non-small cell lung cancer. CD133 is expressed by stemcells and has been described as an indicator for shortened progression-free survival in Lung Cancer patients receiving Cisplatin treatment. N-Cadherin seems to be a marker for epithelial-mesenchymal-transition, a process that also may indicate limited response to therapy. In association to this c-Met is pictured as a stem cell marker. Furthermore c-Met plays a role in the epithelial-mesenchymal transition during cancer invasion.

One patient (*1953), was diagnosed with metastatic pulmonary adenocarcinoma (cT1a cN0 cM1a, stage IV) in 06, 2010. Molecular pathology resulted in EGFR and KRAS wildtype and high c-Met overexpression (4, 90%). First-line treatment consisted of 4 cycles CisPem till 09/10 resulting in partial response. After 8 months watch&wait the patient progressed and received Erlotinib starting June 2011 for 6 months. Due to progression the treatment was switched to a re-challenge with CisPem administered from 09/11-03/12 resulting again in partial response. During CisPem treatment patients' blood was drawn and examined for circulating tumor cells.

Mononuclearic cells including CTCs were enriched using a Ficoll density gradient followed by depletion of hematopoietic cells employing a mixture of immunomagnetic beads linked with monoclonal CD45 and CD15 antibodies. The resulting cell suspension was subsequently spun on glass slides. Immunofluorescence double labeling with primary antibodies against CD45 was utilized to identify hematopoietic cells. Pan-CK, CD133 and N-Cadherin were taken as markers for circulating tumor cell subgroups. N-Cadherin and CD133+ were examined on different slides of the same patient at the same timepoint due to practical reasons. Furthemore cells were stained with DAPI to identify the nucleus. An average number of 2 CD133+ cells and 2 N-Cadherin+ cells plus 17 respectively 22 Pan-CK+ cells per 10 visual fields (vf) in 20x magnification on each slide (1360 vf/slide) was found.

Cortes-Dericks and colleagues examined mesothelioma cell lines with stem cell characteristics and found those to be resistant to Cisplatin and Pemetrexed treatment. They proposed a different approach in those cases since the remaining CD133+ cells may be the origin of relapsing tumors. Gottschling and others analyzed pre-therapeutic tissue specimens of patients with a long-term response ( 3 years; LTR) to gefitinib treated in the IRESSA expanded access program (EAP) for molecular markers. They found that 40% of the patients with a LTR had CD133+ expression and small molecules might therefore be efficient in this cohort.

In conclusion this example supports CTC subgroups as potential biomarkers of an individual therapeutic approach. The identified stem cells are in accordance with a c-Met overexpressing primary tumor. Many solid tumors consist of cells with different molecular characteristics. Certain cells like for instance stem cells or mesenchymal cells may distinctively be selected during the treatment of first choice and lead to relapse. An approach relying solely on the molecular characterization of the primary tumor upfront may be insufficient.

### Table 1: Patient demographics for quantification

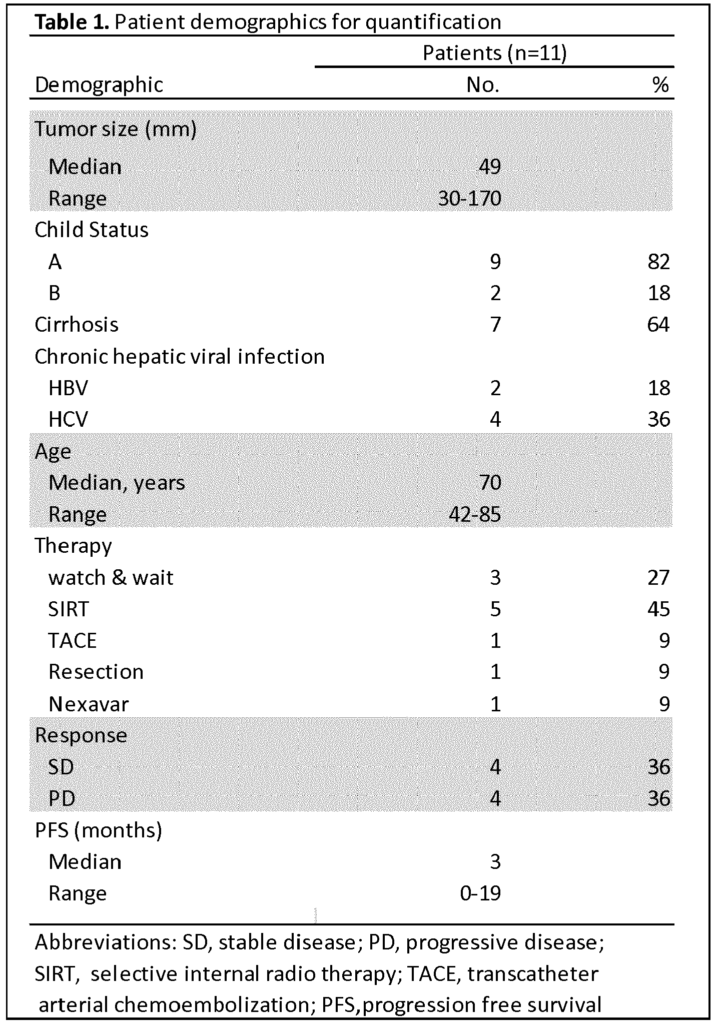

### Table 2: Patient demographics for qRT-PCR

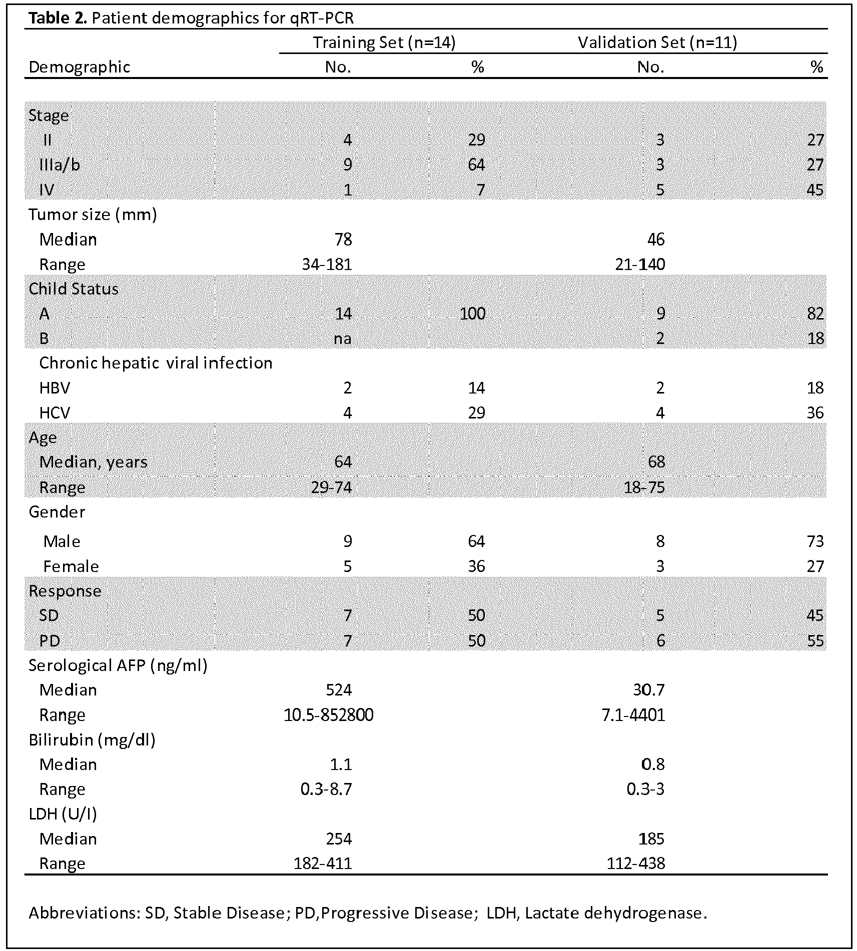

### Table 3: Linear multivariate regression models

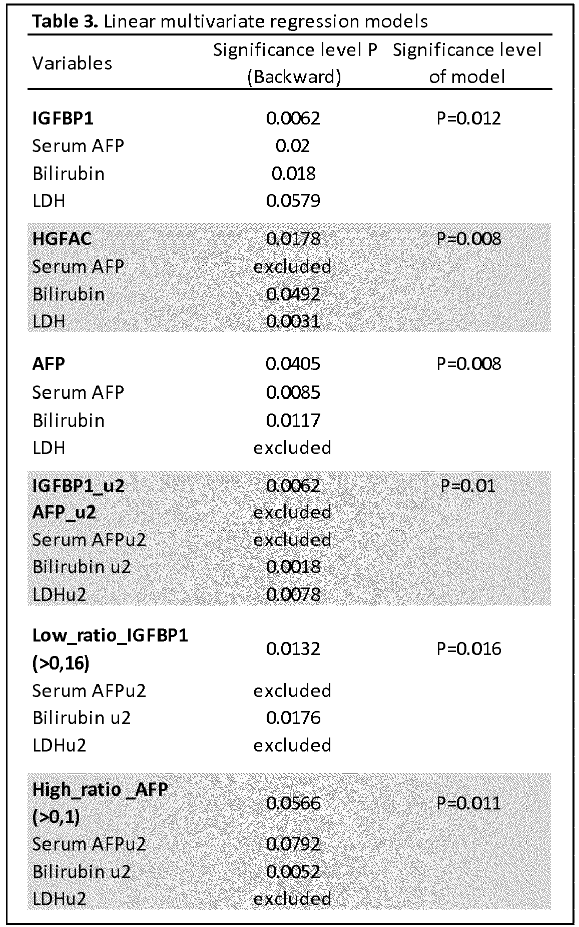

### Literature:

1. Llovet JM, Burroughs A, Bruix J. Hepatocellular carcinoma. Lancet 2003;362:1907-1917.
2. Jemal A, Bray F, Center MM, Ferlay J, Ward E, Forman D. Global cancer statistics. CA Cancer J Clin 2011;61:69-90.
3. Belghiti J, Panis Y, Farges O, Benhamou JP, Fekete F. Intrahepatic recurrence after resection of hepatocellular carcinoma complicating cirrhosis. Ann Surg 1991;214:114-117.
4. Bruix J, Sherman M. Management of hepatocellular carcinoma. Hepatology 2005;42:1208-1236.
5. Lai Q, Avolio AW, Lerut J, Singh G, Chan SC, Berloco PB, Tisone G, et al. Recurrence of Hepatocellular Cancer after Liver Transplantation: The Role of Primary Resection and of Salvage Transplantation in East and West. J Hepatol 2012.
6. Toso C, Cader S, Mentha-Dugerdil A, Meeberg G, Majno P, Morard I, Giostra E, et al. Factors predicting survival after post-transplant hepatocellular carcinoma recurrence. J Hepatobiliary Pancreat Sci 2012.
7. Sangro B, Bilbao JI, Boan J, Martinez-Cuesta A, Benito A, Rodriguez J, Panizo A, et al. Radioembolization using 90Y-resin microspheres for patients with advanced hepatocellular carcinoma. Int J Radiat Oncol Biol Phys 2006;66:792-800.
8. Lau WY, Lai EC, Leung TW. Current role of selective internal irradiation with yttrium-90 microspheres in the management of hepatocellular carcinoma: a systematic review. Int J Radiat Oncol Biol Phys 2011;81:460-467.
9. Sun YF, Yang XR, Zhou J, Qiu SJ, Fan J, Xu Y. Circulating tumor cells: advances in detection methods, biological issues, and clinical relevance. J Cancer Res Clin Oncol 2011;137:1151-1173.
10. Lianidou ES, Markou A. Circulating tumor cells in breast cancer: detection systems, molecular characterization, and future challenges. Clin Chem 2011;57:1242-1255.
11. Hou JM, Krebs M, Ward T, Sloane R, Priest L, Hughes A, Clack G, et al. Circulating tumor cells as a window on metastasis biology in lung cancer. Am J Pathol 2011;178:989-996.
12. Krebs MG, Sloane R, Priest L, Lancashire L, Hou JM, Greystoke A, Ward TH, et al. Evaluation and prognostic significance of circulating tumor cells in patients with non-small-cell lung cancer. Journal of clinical oncology : official journal of the American Society of Clinical Oncology 2011;29:1556-1563.
13. Waguri N, Suda T, Nomoto M, Kawai H, Mita Y, Kuroiwa T, Igarashi M, et al. Sensitive and specific detection of circulating cancer cells in patients with hepatocellular carcinoma; detection of human telomerase reverse transcriptase messenger RNA after immunomagnetic separation. Clin Cancer Res 2003;9:3004-3011.
14. Xu W, Cao L, Chen L, Li J, Zhang XF, Qian HH, Kang XY, et al. Isolation of circulating tumor cells in patients with hepatocellular carcinoma using a novel cell separation strategy. Clin Cancer Res 2011;17:3783-3793.
15. Vona G, Estepa L, Beroud C, Damotte D, Capron F, Nalpas B, Mineur A, et al. Impact of cytomorphological detection of circulating tumor cells in patients with liver cancer. Hepatology 2004;39:792-797.
16. Guo J, Yao F, Lou Y, Xu C, Xiao B, Zhou W, Chen J, et al. Detecting carcinoma cells in peripheral blood of patients with hepatocellular carcinoma by immunomagnetic beads and rt-PCR. J Clin Gastroenterol 2007;41:783-788.
17. Schulze K, Beneken C, Staufer K, Nashan B, Lohse AW, Pantel K, Riethdorf S, et al. Detection of Epcam-Positive Circulating Tumor Cells in Patients with Hepatocellular Carcinoma - a Pilot Study. Hepatology 2011;54:1357a-1357a.
18. Moldenhauer G, Momburg F, Moller P, Schwartz R, Hammerling GJ. Epithelium-specific surface glycoprotein of Mr 34,000 is a widely distributed human carcinoma marker. Br J Cancer 1987;56:714-721.
19. Cristofanilli M, Budd GT, Ellis MJ, Stopeck A, Matera J, Miller MC, Reuben JM, et al. Circulating tumor cells, disease progression, and survival in metastatic breast cancer. N Engl J Med 2004;351:781-791.
20. Maheswaran S, Sequist LV, Nagrath S, Ulkus L, Brannigan B, Collura CV, Inserra E, et al. Detection of mutations in EGFR in circulating lung-cancer cells. N Engl J Med 2008;359:366-377.
21. Allard WJ, Matera J, Miller MC, Repollet M, Connelly MC, Rao C, Tibbe AG, et al. Tumor cells circulate in the peripheral blood of all major carcinomas but not in healthy subjects or patients with nonmalignant diseases. Clin Cancer Res 2004;10:6897-6904.
22. Paterlini-Brechot P, Benali NL. Circulating tumor cells (CTC) detection: clinical impact and future directions. Cancer Lett 2007;253:180-204.
23. Kalluri R, Weinberg RA. The basics of epithelial-mesenchymal transition. J Clin Invest 2009;119:1420-1428.
24. Yu M, Stott S, Toner M, Maheswaran S, Haber DA. Circulating tumor cells: approaches to isolation and characterization. J Cell Biol 2011;192:373-382.
25. Gradilone A, Raimondi C, Nicolazzo C, Petracca A, Gandini O, Vincenzi B, Naso G, et al. Circulating tumour cells lacking cytokeratin in breast cancer: the importance of being mesenchymal. J Cell Mol Med 2011;15:1066-1070.
26. Raimondi C, Gradilone A, Naso G, Vincenzi B, Petracca A, Nicolazzo C, Palazzo A, et al. Epithelial-mesenchymal transition and stemness features in circulating tumor cells from breast cancer patients. Breast Cancer Res Treat 2011;130:449-455.
27. Joly E, Hudrisier D. What is trogocytosis and what is its purpose? Nat Immunol 2003;4:815.
28. Marrinucci D, Bethel K, Bruce RH, Curry DN, Hsieh B, Humphrey M, Krivacic RT, et al. Case study of the morphologic variation of circulating tumor cells. Hum Pathol 2007;38:514-519.
29. Hofman V, Ilie MI, Long E, Selva E, Bonnetaud C, Molina T, Venissac N, et al. Detection of circulating tumor cells as a prognostic factor in patients undergoing radical surgery for non-small-cell lung carcinoma: comparison of the efficacy of the CellSearch Assay and the isolation by size of epithelial tumor cell method. Int J Cancer 2011;129:1651-1660.
30. Zhou L, Liu J, Luo F. Serum tumor markers for detection of hepatocellular carcinoma. World Journal of Gastroenterology 2006;12:1175-1181.
31. Gong Y, Cui L, Minuk GY. The expression of insulin-like growth factor binding proteins in human hepatocellular carcinoma. Mol Cell Biochem 2000;207:101-104.

## Claims

1. A method for identifying one or more subgroup(s) of circulating tumor cells (CTC) in a population of CTCs or in a sample comprising the steps
a) depleting hematopoietic cells from the population of CTCs or the sample,
b) testing the obtained cell suspension for the presence of one or more epithel marker(s),
c) testing the obtained cell suspension for the presence of one or more mesenchymal marker(s).

2. A method according to claim 1, comprising the step of isolating or enrichment of peripheral blood mononuclear cells (PBMC) from the population of CTCs or the sample.

3. A method according to claim 1 or 2, wherein PBMCs are isolated or enriched by density gradient centrifugation.

4. A method according to claim 1 to 3, wherein hematopoietic cells are depleted using anti-CD45 antibodies, for example immunomagnetic beads coated with anti-CD45 antibodies.

5. A method according to claim 1 to 4 wherein 55 to 85 %, preferably 65 to 75 %, most preferred about 70 % of the hematopoietic cells are depleted from the population of CTCs or the sample.

6. A method according to claim 1 to 5 comprising the step of enrichment of epithelial cells after the CD45-depletion step, for example with anti-EpCAM (immunomagnetic) beads.

7. A method according to claim 1 to 6 wherein the epithel marker(s) is/are selected from the group comprising epithelial cell surface markers, epithelial cell adhesion molecule (EpCAM), epithelial growth factor recepctor (EGFR), cytokeratin (CK), pan-cytokeratin (Pan-CK), Hep-Par-1, cytoplasmatic intermediate filament protein (Krt18), ASGPR1, DAPI, AFP, IGFBP1, CD133, LTR.

8. A method according to claim 1 to 7 wherein the mesenchymal marker(s) is / are selected from the group comprising N-Cadherin and Vimentin.

9. A method according to claim 1 to 8 comprising the step of testing the obtained cell suspension for the presence of one or more marker(s) specific for a tumor and / or a particular tissue.

10. A method according to claim 1 to 9 further comprising the step of testing the obtained cell suspension for one or more marker(s) for epithelial-to-mesenchymal-transition (EMT), for example for N-Cadherin and/ or c-Met.

11. A method according to claim 1 to 10 wherein the presence of the marker(s) in the CTC subgroup is/are detected by immunofluorescence, PCR or FISH.

12. Use of the method according to claims 1 to 11 to determine the ratio of one subgroup of CTCs to another subgroup of CTCs in the CTC population or the sample, for example to determine the ratio of mesenchymal CTCs to epithelial CTCs in the CTC population or the sample.

13. Use of the method according to claim 1 to 11 for diagnosis of tumor, for monitoring of tumor development, for prognosis, for evaluation of treatment, for monitoring of treatment, for disease control, for determination of the stage of epithelial-to-mesenchymal-transition (EMT), for characterization of the tumor type, for hepatocellular carcinoma (HCC) and / or non-small cell lung cancer, for the identification and / or validation of pharmaceutical coumpounds.

14. Diagnostic device or test kit for the identification and / or characterization of one or more subgroup(s) of CTCs in a CTC population or a sample or for determination of the ratio of different subgroups of CTCs in a CTC population or a sample.

15. Diagnostic device or test kit comprising means for depleting hematopoietic cells from a population of CTCs or a sample and means for detection and / or identification of one or more marker(s) selected from the group of epithelial markers, mesenchymal markers and / or tumor markers in the CTC population or the sample and means for the visualization of CTC subgroups.
